# EUROPÄISCHE PATENTSCHRIFT

(11) **EP 1 917 228 B1**
(45) Veröffentlichungstag und Bekanntmachung des Hinweises auf die Patenterteilung: **24.11.2010**
(21) Anmeldenummer: 06792831.7
(22) Anmeldetag: 16.08.2006
(51) Int. Cl.: C07C 51/41, C07F 5/06, C01B 3/00

(54) **MESOPORÖSES METALLORGANISCHES GERÜSTMATERIAL**
MESOPOROUS METAL-ORGANIC FRAMEWORK
MATERIAU MESOPOREUX A STRUCTURE ORGANOMETALLIQUE

(30) Priorität: 22.08.2005 DE 102005039654
(43) Veröffentlichungstag der Anmeldung: 07.05.2008
(73) Patentinhaber: BASF SE, 67056 Ludwigshafen (DE)
(72) Erfinder: SCHUBERT, Markus, 67063 Ludwigshafen (DE); MÜLLER, Ulrich, 67435 Neustadt (DE); MATTENHEIMER, Hendrick, 67063 Ludwigshafen (DE); TONIGOLD, Markus, 89134 Blaustein (DE)
(86) Internationale Anmeldenummer: PCT/EP2006/065346
(87) Internationale Veröffentlichungsnummer: WO 2007/023119

(56) Entgegenhaltungen:
- WO-A-99/05151
- JP-A- 59 155 333
- US-A- 5 648 508

## Beschreibung

Die vorliegende Erfindung betrifft ein poröses metallorganisches Gerüstmaterial sowie ein Verfahren zu dessen Herstellung und dessen Verwendung.

Poröse metallorganische Gerüstmaterialien sind im Stand der Technik bekannt.

Diese enthalten typischerweise mindestens eine an mindestens ein Metallion koordinativ gebundene mindestens zweizähnige organische Verbindung. Solche metallorganischen Gerüstmaterialien (MOF = metal organic framework) werden beispielsweise in US-A 5,648,508, EP-A 0 790 253, M. O. Keefe, J. Sol. State Chem. 152 (2000), 3-20; H. Li et al., Nature 402 (1999), 276; M. Eddaoudi, Topics in Catalysis 9 (1999), 105-111; B. Chen et al., Science 291 (2001), 1021 - 1023 und DE-A 101 11 230 beschrieben.

Die im Stand der Technik untersuchten metallorganischen Gerüstmaterialien eignen sich in vielfältiger Weise. Diese können beispielsweise zur Speicherung, zur Abtrennung oder bei der chemischen Umsetzung von Stoffen eingesetzt werden. Insbesondere können diese als Träger für Katalysatoren oder Katalysatoren verwendet werden.

Auf der Suche nach weiteren Anwendungsgebieten oder Verbesserung der Eigenschaften solcher metallorganischer Gerüstmaterialien sind zahlreiche Kombinationen von Metallionen und mindestens zweizähniger organischer Verbindungen getestet worden. Hierbei gelangten in jüngerer Zeit auch metallorganische Gerüstmaterialien in den Blickpunkt, bei denen es sich bei dem Metallion um ein Hauptgruppenelement des Periodensystems handelt.

T. Loiseau et al., Chem. Eur. J. 10 (2004), 1373-1382 beschreiben beispielsweise poröses Aluminiumterephthalat. Dieses weist jedoch Poren vergleichsweise geringer Dimension auf. Diese liegt in einem Bereich, wie sie auch für andere metallorganische Gerüstmaterialien auf Basis anderer Metalle, wie etwa Zink, bekannt und üblich sind. Dies ist insbesondere nachteilig für Reaktionen, bei denen vergleichsweise großvolumige Reaktanden in einer diffusionslimitierten Reaktion umgesetzt werden.

Z.-Z. Lin et al., Eur. J. Inorg. Chem. 2005, 77-81 beschreiben ein Gerüstmaterial basierend auf In^{III}-BTC (BTC = 1,3,5-Benzoltricarbonsäure). Das so erhaltene Gerüstmaterial weist jedoch geringe bis keine Absorptionseigenschaften auf. Dies ist insbesondere nachteilig für Anwendungen auf dem Gebiet der Trennung oder Speicherung von Verbindungen, insbesondere Gasen.

Es besteht also nach wie vor ein Bedarf, neue metallorganische Gerüstmaterialien bereitzustellen, die zumindest teilweise überlegene Eigenschaften gegenüber den im Stand der Technik bekannten Gerüstmaterialien aufweisen.

Eine Aufgabe der vorliegenden Erfindung liegt somit darin, ein poröses metallorganisches Gerüstmaterial bereitzustellen, das die oben geschilderten Eigenschaften aufweist.

Die Aufgabe wird gelöst durch ein poröses metallorganisches Gerüstmaterial enthaltend mindestens eine an mindestens ein Metallion koordinativ gebundene mindestens zweizähnige organische Verbindung, wobei das mindestens eine Metallion Al^{III} ist und es sich bei der mindestens einen mindestens zweizähnigen organischen Verbindung um einen sechsgliedrigen aromatischen Kohlenwasserstoffring A, bei dem ein oder mehrere Ringkohlenstoffatome durch Stickstoff ersetzt sein können und der drei Substituenten X sowie gegebenenfalls einen oder mehrere Substituenten ausgewählt aus der Gruppe bestehend aus R, NRR', OR, SR, F, Cl, und Br aufweist, wobei R, R' unabhängig voneinander Wasserstoff, optional mit einem oder mehreren Fluoratomen substituiertes Methyl oder optional mit einem oder mehreren Fluoratomen substituiertes Ethyl sind und jedes X jeweils unabhängig C(=O)O⁻, C(=S)O⁻, C(=O)S⁻, C(=S)S⁻ oder deren protonierte Form ist, handelt, wobei das metallorganische Gerüstmaterial in Pulverform eine spezifische Oberfläche nach Langmuir von mindestens 800 m²/g aufweist und wobei mindestens 50% des Poremvolumens durch Poren mit einem Porendurchmesser von 2 bis 10 nm gebildet wird.

Es wurde nämlich gefunden, dass oben beschriebene Gerüstmaterialien über einen vergleichsweise ungewöhnlich hohen Anteil an großvolumigen Poren (Mesoporen) aufweisen und ebenso vergleichsweise hohe spezifische Oberflächen zeigen, was zu Vorteilen bei den nachfolgend aufgeführten Verwendungen führt. Dies ist umso überraschender, da diese Eigenschaften nicht bestehen bleiben, wenn Aluminium durch sein Gruppenanalogon im Periodensystem Indium ausgetauscht wird oder anstelle der mit drei X-Gruppen substituierten organischen Verbindung nur zwei dieser Gruppen für den Aufbau eines metallorganischen Gerüstmaterials eingesetzt werden. Dies gilt insbesondere, wenn es sich bei der organischen Verbindung um ein Benzoltricarboxylat handelt.

Darüber hinaus zeigen die Röntgendiffraktogramme des erfindungsgemäßen Gerüstmaterials im Vergleich zu den oben genannten im Stand der Technik bekannten Gerüstmaterialien kaum die für bekannte Gerüstmaterialien charakteristischen scharfen Signale zwischen 5 und 30° (2 Θ), insbesondere zwischen 5 und 12°.

Das metallorganische Gerüstmaterial enthält mindestens eine mindestens zweizähnige organische Verbindung, wobei es sich bei der mindestens einen mindestens zweizähnigen organischen Verbindung um einen sechsgliedrigen aromatischen Kohlenwasserstoffring A, bei dem ein oder mehrere Ringkohlenstoffatome durch Stickstoff ersetzt sein können und der drei Substituenten X sowie gegebenenfalls einen oder mehrere Substituenten ausgewählt aus der Gruppe bestehend aus R, NRR', OR, SR, F, Cl, und Br aufweist, wobei R, R' unabhängig voneinander Wasserstoff, optional mit einem oder mehreren Fluoratomen substituiertes Methyl oder optional mit einem oder mehreren Fluoratomen substituiertes Ethyl sind und jedes X jeweils unabhängig C(=O)O⁻, C(=S)O⁻, C(=O)S⁻, C(=S)S⁻ oder deren protonierte Form ist, handelt. Es können in dem Gerüstmaterial auch weitere organische Verbindungen auftreten, insbesondere eine oder mehrere einzähnige Liganden. Hierbei ist jedoch bevorzugt, dass der molare Anteil an erstgenannter organischer Verbindung, die vorhanden sein muss, mindestens 50 %, mehr bevorzugt mindestens 75 % und besonders bevorzugt mindestens 90 % der Gesamtmenge der organischen Verbindungen im Gerüstmaterial beträgt.

Vorzugsweise handelt es sich bei dem Ring A um einen Benzol-, Pyridin-, Pyridazin-, Pyrimidin-, Pyrazin- oder Triazinring. Besonders bevorzugt handelt es sich bei A um Benzol.

Weiterhin weist der Ring A drei Substituenten X auf. Hierbei handelt es sich um die Carboxylatgruppe sowie deren Thioanaloga. Zumindest ein Teil der im Gerüstmaterial an A auftretenden Carboxylatgruppen und/oder Thioanaloga können in protonierten Form vorliegen.

Zudem kann der Ring A einen oder mehrere weitere Substituenten aufweisen. Hierbei handelt es sich um die funktionellen Gruppen R, NRR', OR, SR, F, Cl, und Br, wobei R und R' unabhängig voneinander Wasserstoff, Methyl oder Ethyl sein können. Die Methyl- sowie die Ethylgruppe können auch ein- oder mehrfach fluoriert sein. Vorzugsweise weist der Ring A jedoch mit Ausnahme von X keine weiteren Substituenten auf.

Besonders bevorzugt handelt es sich bei der organischen Verbindung um 1,2,3-, 1,2,4-oder 1,3,5-Benzoltricarboxylat bzw. deren zumindest teilweise protonierten Analoga. Insbesondere bevorzugt ist 1,3,5-Benzoltricarboxylat.

Das erfindungsgemäße metallorganische Gerüstmaterial enthält Poren, insbesondere Mikro- und/oder Mesoporen. Mikroporen sind definiert als solche mit einem Durchmesser von 2 nm oder kleiner und Mesoporen sind definiert durch einen Durchmesser im Bereich von 2 bis 50 nm, jeweils entsprechend nach der Definition, wie sie in Pure Applied Chemistry 57 (1985), Seiten 603 - 619, insbesondere auf Seite 606 angegeben ist. Die Anwesenheit von Mikro- und/oder Mesoporen kann mit Hilfe von Sorptionsmessungen überprüft werden, wobei diese Messungen die Aufnahmekapazität der metallorganischen Gerüstmaterialien für Stickstoff bei 77 K gemäß DIN 66131 und/oder DIN 66134 bestimmt.

Das erfindungsgemäße metallorganische Gerüstmaterial besitzt einen durchschnittlichen Porendurchmesser von 2 bis 10 nm, bevorzugt von 3 bis 9 nm. Insbesondere ist bevorzugt, dass die Verteilung der Porengröße maximal +/- 5 nm beträgt. Weiterhin bevorzugt liegt der am häufigsten auftretende Porendurchmesser in einem Bereich von 3 bis 9 nm.

Die spezifische Oberfläche - berechnet nach dem Langmuir-Modell gemäß DIN 66135 (DIN 66131, 66134) beträgt für ein erfindungsgemäßes metallorganisches Gerüstmaterial in Pulverform mindestens 800 m²/g. Bevorzugt beträgt die spezifische Oberfläche mindestens 1000 m²/g und insbesondere bevorzugt mindestens 1300 m²/g. Ganz besonders bevorzugt beträgt die spezifische Oberfläche mindestens 1500 m²/g.

Hierbei ist bevorzugt, dass mindestens 50 % des Porenvolumens durch Poren mit einem Porendurchmesser von 2 bis 10 nm (Mesoporen) gebildet werden. Vorzugsweise beträgt der Anteil am Porenvolumen mindestens 65 %, ganz besonders bevorzugt mindestens 80 %. Das Porenvolumen beträgt vorzugsweise mindestens 0,8 ml/g, mehr bevorzugt mindestens 1,1 ml/g.

MOF-Formkörper können eine niedrige spezifische Oberfläche besitzen. Vorzugsweise beträgt diese jedoch mehr als 10 m²/g, mehr bevorzugt mehr als 50 m²/g und weiter mehr bevorzugt mehr als 500 m²/g.

Für das erfindungsgemäße metallorganische Gerüstmaterial treten neben den oben beschriebenen Poren in einem Formkörper auch größere Poren auf, deren Größenverteilung variieren kann. Vorzugsweise wird jedoch mehr als 50 % des gesamten Porenvolumens, insbesondere mehr als 75 % von Poren mit einem Porendurchmesser von bis zu 1000 nm gebildet. Vorzugsweise wird jedoch ein Großteil des Porenvolumens von Poren aus zwei Durchmesserbereichen gebildet. Es ist daher weiter bevorzugt, wenn mehr als 25 % des gesamten Porenvolumens, insbesondere mehr als 50 % des gesamten Porenvolumens von Poren gebildet wird, die in einem Durchmesserbereich von 100 nm bis 800 nm liegen und wenn mehr als 15 % des gesamten Porenvolumens, insbesondere mehr als 25 % des gesamten Porenvolumens von Poren gebildet wird, die in einem Durchmesserbereich bis zu 10 nm liegen. Die Porenverteilung kann mittels Quecksilber-Porosymmetrie bestimmt werden.

Ein weiterer Gegenstand der vorliegenden Erfindung ist ein Verfahren zur Herstellung eines erfindungsgemäßen Gerüstmaterials den Schritt enthaltend

Umsetzung mindestens einer Metallverbindung mit mindestens einer mindestens zweizähnigen organischen Verbindung, die koordinativ an das Metallion binden kann, in N,N-Dimethylformamid, N,N-Diethylformamid, N-Methylpyrrolidon oder Mischungen davon, wobei das Metall Al^{III} ist und wobei es sich bei der mindestens einen mindestens zweizähnigen organischen Verbindung um einen sechsgliedrigen aromatischen Kohlenwasserstoffring A, bei dem ein oder mehrere Ringkohlenstoffatome durch Stickstoff ersetzt sein können und der drei Substituenten X sowie gegebenenfalls einen oder mehrere Substituenten ausgewählt aus der Gruppe bestehend aus R, NRR', OR, SR, F, Cl, und Br aufweist, wobei R, R' unabhängig voneinander Wasserstoff, optional mit einem oder mehreren Fluoratomen substituiertes Methyl oder optional mit einem oder mehreren Fluoratomen substituiertes Ethyl sind und jedes X jeweils unabhängig C(=O)O⁻, C(=S)O⁻, C(=O)S⁻, C(=S)S⁻ oder deren protonierte Form ist, handelt.

Vorzugsweise findet die Umsetzung unter Rühren bei einem Druck von höchsten 2 bar (absolut) statt.

Mehr bevorzugt beträgt der Druck maximal 1230 mbar (absolut). Weiter bevorzugt findet die Umsetzung unter Atmosphärendruck statt.

Durch Einsatz von N,N-Dimethylformamid, N,N-Diethylformamid, N-Methylpyrrolidon oder Mischungen davon kann auf eine Base verzichtet werden. Ebenso kann aber auch eine Base zugesetzt werden.

Bevorzugte Einsatzstoffe bei Verwendung eines Aluminiumsalzes in der Synthese sind die Hydrate von Aluminiumchlorid oder -nitrat, insbesondere ist das Chlorid bevorzugt.

Weiterhin bevorzugt kann die verwendete Metallverbindung zur Herstellung des erfindungsgemäßen porösen metallorganischen Gerüstmaterials nicht-ionisch sein und/oder das Gegenion zum Al^{III}-Kation kann sich von einem protischen Lösemittel ableiten lassen. Durch die Verwendung einer nicht-ionischen Verbindung kann bei geeigneter Wahl vermieden werden, dass bei der Umsetzung zum porösen metallorganischen Gerüstmaterial das Metall in Form eines Salzes vorliegt und so gegebenenfalls Schwierigkeiten bei der Entfernung des korrespondierenden Anions im Metallsalz vermieden werden können, sofern durch die Metallverbindung bei der Umsetzung keine weiteren störenden Salze erzeugt werden. Stellt das Gegenion ein Lösemittelanion dar, kann dieses bei geeigneter Wahl nach Umsetzung als Lösemittel vorliegen, das gleich dem eingesetzten nicht-wässrigen organischen Lösemittel oder verschieden davon sein kann. In letzterem Fall ist bevorzugt, wenn dieses Lösemittel mit dem nicht-wässrigen organischen Lösemittel zumindest zum Teil mischbar ist.

Solche nicht-ionischen Verbindungen beziehungsweise Gegenionen zum Metallkation, die sich von protischen Lösemitteln ableiten lassen, können beispielsweise Metallalkoholate, beispielsweise Methanolate, Ethanolate, Propanolate, Butanolate sein. Ebenso sind Oxide oder Hydroxide denkbar.

Als nicht-wässrige organische Lösemittel dient N,N-Dimethylformamid (DMF), N,N-Diethylformamid (DEF), N-Methylpyrrolidon (NMP), oder Mischungen davon.

Bevorzugte Lösemittel sind DMF, DEF und NMP. Besonders bevorzugt ist DMF.

Der Begriff "nicht-wässrig" bezieht sich vorzugsweise auf ein Lösemittel, das einen Höchstwassergehalt von 10 Gew.-%, mehr bevorzugt 5 Gew.-%, weiterhin mehr bevorzugt 1 Gew.-%, weiterhin bevorzugt 0,1 Gew.-%, besonders bevorzugt 0,01 Gew.-% bezogen auf das Gesamtgewicht des Lösemittels nicht überschreitet.

Vorzugsweise beträgt der Höchstwassergehalt während der Umsetzung 10 Gew.-%, mehr bevorzugt 5 Gew.-% und weiterhin mehr bevorzugt 1 Gew.-%.

Der Begriff "Lösemittel" betrifft reine Lösemittel sowie Gemische von unterschiedlichen Lösemitteln.

Weiterhin bevorzugt schließt sich an den Verfahrensschritt der Umsetzung der mindestens einen Metallverbindung mit der mindestens einen mindestens zweizähnigen organischen Verbindung ein Calcinierungsschritt an. Die hierbei eingestellte Temperatur beträgt typischerweise mehr als 250 °C, bevorzugt 300 bis 400 °C.

Aufgrund des Calcinierungsschrittes kann der in den Poren befindliche Ligand entfernt werden.

Ergänzend oder alternativ hierzu kann die Entfernung von Ligand aus den Poren des porösen metallorganischen Gerüstmaterials durch die Behandlung des gebildeten Gerüstmaterials mit einem nicht-wässrigen Lösemittel erfolgen. Hierbei wird in einer Art "Extraktionsverfahren" der Ligand entfernt und gegebenenfalls im Gerüstmaterial durch ein Lösemittelmolekül ersetzt. Vorzugsweise findet das Extraktionsverfahren vor einer Calcinierung statt, wenn letztere vorgesehen ist.

Die Behandlung erfolgt vorzugsweise mindestens 30 Minuten und kann typischerweise bis zu zwei Tagen betragen. Dies kann bei Raumtemperatur oder erhöhter Temperatur geschehen. Vorzugsweise erfolgt dies unter erhöhter Temperatur, beispielsweise bei mindestens 40 °C, bevorzugt 60 °C. Weiterhin bevorzugt ist eine Umsetzung bei der Siedetemperatur des Lösemittels (Rückfluss).

Die Behandlung kann in einem einfachen Kessel durch Aufschlämmen und Rühren des Gerüstmaterials erfolgen. Es können auch Extraktionsapparaturen wie Soxhlet-Apparaturen, insbesondere technische Extraktionsapparaturen verwendet werden.

Als geeignete Lösemittel können die oben genannten verwendet werden, also C₁₋₆-Alkanol, DMSO, DMF, DEF, Acetonitril, Toluol, Dioxan, Benzol, Chlorbenzol, MEK, Pyridin, THF, Essigsäureethylester, gegebenenfalls halogeniertes C₁₋₂₀₀-Alkan, Sulfolen, Glykol, NMP oder Mischungen davon.

Das verwendete Lösemittel zur Extraktion kann gleich oder verschieden zu demjenigen für die Umsetzung der mindestens einen Metallverbindung mit der mindestens einen mindestens zweizähnigen organischen Verbindung sein. Insbesondere ist es bei der Extraktion nicht unbedingt erforderlich, dass das Lösemittel wasserfrei ist.

Ein bevorzugtes Extraktionslösemittel ist Methanol, Ethanol, Aceton, MEK oder eine Mischung davon. Besonders bevorzugt ist Methanol.

Ein weiterer Gegenstand der vorliegenden Erfindung ist ein Verfahren zur Herstellung des erfindungsgemäßen metallorganischen Gerüstmaterials, bei dem Al^{III} durch anodische Oxidation für die Umsetzung bereitgestellt wird.

Die Herstellung von metallorganischen Gerüstmaterialien auf elektrochemischem Wege ist in WO-A 2005/049892 beschrieben.

Das erfindungsgemäße metallorganische Gerüstmaterial kann pulverförmig beziehungsweise als Agglomerat vorliegen. Das Gerüstmaterial kann als solches verwendet werden oder es wird in einen Formkörper umgewandelt. Bevorzugte Verfahren sind hierbei die Verstrangung oder Tablettierung. Bei der Formkörperherstellung kann das Gerüstmaterial weitere Materialien, wie beispielsweise Binder, Gleitmittel oder andere Additive aufweisen, welche während der Herstellung hinzugesetzt werden. Ebenso ist es denkbar, dass das Gerüstmaterial weitere Bestandteile aufweist, wie zum Beispiel Adsorbentien wie Aktivkohle oder dergleichen.

Hinsichtlich der möglichen Geometrien der Formkörper existieren im Wesentlichen keine Beschränkungen. Beispielsweise sind unter anderem Pellets wie beispielsweise scheibenförmige Pellets, Pillen, Kugeln, Granulat, Extrudate wie beispielsweise Stränge, Waben, Gitter oder Hohlkörper zu nennen.

Zur Herstellung dieser Formkörper sind grundsätzlich sämtliche geeigneten Verfahren möglich. Es sind insbesondere folgende Verfahrensführungen bevorzugt:
- Kneten/Kollern des Gerüstmaterials allein oder zusammen mit mindestens einem Bindemittel und/oder mindestens einem Anteigungsmittel und/oder mindestens einer Templatverbindung unter Erhalt eines Gemisches; Verformen des erhaltenen Gemisches mittels mindestens einer geeigneten Methode wie beispielsweise Extrudieren; optional Waschen und/oder Trocknen und/oder Calcinieren des Extrudates; optional Konfektionieren.
- Aufbringen des Gerüstmaterials auf mindestens ein gegebenenfalls poröses Trägermaterial. Das erhaltene Material kann dann gemäß der vorstehend beschriebenen Methode zu einem Formkörper weiterverarbeitet werden.
- Aufbringen des Gerüstmaterials auf mindestens ein gegebenenfalls poröses Substrat.

Kneten/Kollern und Verformen kann gemäß eines jeden geeigneten Verfahrens erfolgen, wie beispielsweise in Ullmanns Enzyklopädie der Technischen Chemie, 4. Auflage, Band 2, S. 313 ff. (1972) beschrieben.

Beispielsweise kann das Kneten/Kollern und/oder Verformen mittels einer Kolbenpresse, Walzenpresse in Anwesenheit oder Abwesenheit mindestens eines Bindermaterials, Compoundieren, Pelletieren, Tablettieren, Extrudieren, Co-Extrudieren, Verschäumen, Verspinnen, Beschichten, Granulieren, bevorzugt Sprühgranulieren, Versprühen, Sprühtrocknen oder einer Kombination aus zwei oder mehr dieser Methoden erfolgen.

Ganz besonders bevorzugt werden Pellets und/oder Tabletten hergestellt.

Das Kneten und/oder Verformen kann bei erhöhten Temperaturen wie beispielsweise im Bereich von Raumtemperatur bis 300 °C und/oder bei erhöhtem Druck wie beispielsweise im Bereich von Normaldruck bis hin zu einigen hundert bar und/oder in einer Schutzgasatmosphäre wie beispielsweise in Anwesenheit mindestens eines Edelgases, Stickstoff oder einem Gemisch aus zwei oder mehr davon erfolgen.

Das Kneten und/oder Verformen wird gemäß einer weiteren Ausführungsform unter Zugabe mindestens eines Bindemittels durchgeführt, wobei als Bindemittel grundsätzlich jede chemische Verbindung eingesetzt werden kann, die die zum Kneten und/oder Verformen gewünschte Viskosität der zu verknetenden und/oder verformenden Masse gewährleistet. Demgemäß können Bindemittel im Sinne der vorliegenden Erfindung sowohl viskositätserhöhende als auch viskositätserniedrigende Verbindungen sein.

Als unter anderem bevorzugte Bindemittel sind beispielsweise Aluminiumoxid oder Aluminiumoxid enthaltende Binder, wie sie beispielsweise in WO 94/29408 beschrieben sind, Siliciumdioxid, wie es beispielsweise in EP 0 592 050 A1 beschrieben ist, Mischungen ais Siliciumdioxid und Aluminiumoxid, wie sie beispielsweise in WO 94/13584 beschrieben sind, Tonminerale, wie sie beispielsweise in JP 03-037156 A beschrieben sind, beispielsweise Montmorillonit, Kaolin, Bentonit, Hallosit, Dickit, Nacrit und Anauxit, Alkoxysilane, wie sie beispielsweise in EP 0 102 544 B1 beschrieben sind, beispielsweise Tetraalkoxysilane wie beispielsweise Tetramethoxysilan, Tetraethoxysilan, Tetrapropoxysilan, Tetrabutoxysilan, oder beispielsweise Trialkoxysilane wie beispielsweise Trimethoxysilan, Triethoxysilan, Tripropoxysilan, Tributoxysilan, Alkoxytitanate, beispielsweise Tetraalkoxytitanate wie beispielsweise Tetramethoxytitanat, Tetraethoxytitanat, Tetrapropoxytitanat, Tetrabutoxytitanat, oder beispielsweise Trialkoxytitanate wie beispielsweise Trimethoxytitanat, Triethoxytitanat, Tripropoxytitanat, Tributoxytitanat, Alkoxyzirkonate, beispielsweise Tetraalkoxyzirkonate wie beispielsweise Tetramethoxyzirkonat, Tetraethoxyzirkonat, Tetrapropoxyzirkonat, Tetrabutoxyzirkonat oder beispielsweise Trialkoxyzirkonate wie beispielsweise Trimethoxyzirkonat, Triethoxyzirkonat, Tripropoxyzirkonat, Tributoxyzirkonat, Silikasole, amphiphile Substanzen und/oder Graphite zu nennen. Insbesondere bevorzugt ist Graphit.

Als viskositätssteigernde Verbindung kann beispielsweise auch, gegebenenfalls zusätzlich, zu den oben genannten Verbindungen eine organische Verbindung und/oder ein hydrophiles Polymer wie beispielsweise Cellulose oder ein Cellulosederivat wie beispielsweise Methylcellulose und/oder ein Polyacrylat und/oder ein Polymethacrylat und/oder ein Polyvinylalkohol und/oder ein Polyvinylpyrrolidon und/oder ein Polyisobuten und/oder ein Polytetrahydrofuran und/oder ein Polyethylenoxid eingesetzt werden.

Als Anteigungsmittel kann unter anderem bevorzugt Wasser oder mindestens ein Alkohol wie beispielsweise ein Monoalkohol mit 1 bis 4 C-Atomen wie beispielsweise Methanol, Ethanol, n-Propanol, iso-Propanol, 1-Butanol, 2-Butanol, 2-Methyl-1-propanol oder 2-Methyl-2-propanol oder ein Gemisch aus Wasser und mindestens einem der genannten Alkohole oder ein mehrwertiger Alkohol wie beispielsweise ein Glykol, bevorzugt ein wassermischbarer mehrwertiger Alkohol allein oder als Gemisch mit Wasser und/oder mindestens einem der genannten einwertigen Alkohole eingesetzt werden.

Weitere Additive, die zum Kneten und/oder Verformen eingesetzt werden können, sind unter anderem Amine oder Aminderivate wie beispielsweise Tetraalkylammonium-Verbindungen oder Aminoalkohole und Carbonat enthaltende Verbindungen wie etwa Calciumcarbonat. Solche weiteren Additive sind etwa in EP 0 389 041 A1, EP 0 200 260 A1 oder WO 95/19222 beschrieben.

Die Reihenfolge der Additive wie Templatverbindung, Binder, Anteigungsmittel, viskositätssteigernde Substanz beim Verformen und Kneten ist grundsätzlich nicht kritisch.

Gemäß einer weiteren bevorzugten Ausführungsform wird der gemäß Kneten und/oder Verformen erhaltene Formkörper mindestens einer Trocknung unterzogen, die im Allgemeinen bei einer Temperatur im Bereich von 25 bis 300 °C, bevorzugt im Bereich von 50 bis 300 °C und besonders bevorzugt im Bereich von 100 bis 300 °C durchgeführt wird. Ebenso ist es möglich, im Vakuum oder unter Schutzgasatmosphäre oder durch Sprühtrocknung zu trocknen.

Gemäß einer besonders bevorzugten Ausführungsform wird im Rahmen dieses Trocknungsvorgangs mindestens eine der als Additive zugesetzten Verbindungen zumindest teilweise aus dem Formkörper entfernt.

Ein weiterer Gegenstand der vorliegenden Erfindung ist die Verwendung des erfindungsgemäßen porösen metallorganischen Gerüstmaterials zur Aufnahme mindestens eines Stoffes zu dessen Speicherung, Abtrennung, kontrollierten Abgabe oder chemischen Umsetzung sowie als Trägermaterial, beispielsweise für Metalle, Metalloxide, Metallsulfide oder andere Gerüststrukturen, und in der Katalyse.

Bei dem mindestens einen Stoff kann es sich um ein Gas oder eine Flüssigkeit handeln. Vorzugsweise handelt es sich bei dem Stoff um ein Gas.

Im Rahmen der vorliegenden Erfindung werden vereinfachend die Begriffe "Gas" und "Flüssigkeit" verwendet, wobei hier jedoch ebenso Gasgemische sowie Flüssigkeitsgemische beziehungsweise flüssige Lösungen unter dem Begriff "Gas" beziehungsweise "Flüssigkeit" zu verstehen sind.

Bevorzugte Gase sind Wasserstoff, Kohlenwasserstoffe, insbesondere Methan, Ethan, Ethen, Acetylen, Propan, n-Butan sowie i-Butan, Kohlenmonoxid, Kohlendioxid, Stickoxide, Sauerstoff, Schwefeloxide, Halogene, halogenierte Kohlenwasserstoffe, NF₃, SF₆, Ammoniak, Borane, Phosphane, Schwefelwasserstoff, Amine, Formaldehyd, E-delgase, insbesondere Helium, Neon, Argon, Krypton sowie Xenon.

Insbesondere bevorzugt ist die Verwendung eines erfindungsgemäßen metallorganischen Gerüstmaterials zur Wasserstoffspeicherung und für die diffusionslimitierte Reaktion mit großvolumigen Reaktanden. Weiterhin bevorzugt ist die Speicherung von vergleichsweise großen Substanzen, die typischerweise in anderen metallorganischen Gerüstmaterialien oder anderen porösen Materialien wie Zeolithe nicht oder nur unzureichend gespeichert werden können. Beispiele hierfür sind Farbstoffe (Pigmente) oder kleiner Proteine - wie z.B. Enzyme.

Die oben erwähnten Reaktionen können Hydrierungen, Oxidationen in flüssiger Phase, Metathese in flüssiger Phase, Alkoxylierungen, Veretherungen, Verseterungen, Alkylierungen, Hydrolysierungen oder vergleichbare Reaktionen sein. Typische große Reaktanden in diffusionslimitierten Reaktionen können beispielsweise Polyole, substituierte Benzole, Farbstoffe oder andere Verbindungen sein.

Bei dem mindestens einen Stoff kann es sich wie oben beschrieben auch um eine Flüssigkeit handeln. Beispiele für eine solche Flüssigkeit sind Desinfektionsmittel, anorganische oder organische Lösemittel, Treibstoffe - insbesondere Benzin oder Diesel-, Hydraulik-, Kühler-, Bremsflüssigkeit oder ein Öl, insbesondere Maschinenöl. Weiterhin kann es sich bei der Flüssigkeit um halogenierte aliphatische oder aromatische, cyclische oder acyclische Kohlenwasserstoffe oder Mischungen davon handeln. Insbesondere kann die Flüssigkeit Aceton, Acetonitril, Anilin, Anisol, Benzol, Benzonitril, Brombenzol, Butanol, tert.-Butanol, Chinolin, Chlorbenzol, Chloroform, Cyclohexan, Diethylenglykol, Diethylether, Dimethylacetamid, Dimethylformamid, Dimethylsulfoxid, Dioxan, Eisessig, Essigsäureanhydrid, Essigsäureethylester, Ethanol, Ethylencarbonat, Ethylendichlorid, Ethylenglykol, Ethylenglykoldimethylether, Formamid, Hexan, Isopropanol, Methanol, Methoxypropanol, 3-Methyl-1-butanol, Methylenchlorid, Methylethylketon, N-Methylformamid, N-Methylpyrrolidon, Nitrobenzol, Nitromethan, Piperidin, Propanol, Propylencarbonat, Pyridin, Schwefelkohlenstoff, Sulfolan, Tetrachlorethen, Tetrachlorkohlenstoff, Tetrahydrofuran, Toluol, 1,1,1-Trichlorethan, Trichlorethylen, Triethylamin, Triethylenglykol, Triglyme, Wasser oder Mischungen hiervon handeln.

Weiterhin kann der mindestens eine Stoff ein Geruchsstoff sein.

Vorzugsweise handelt es sich bei dem Geruchsstoff um eine flüchtige organische oder anorganische Verbindung, die mindestens eines der Elemente Stickstoff, Phosphor, Sauerstoff, Schwefel, Fluor, Chlor, Brom oder Iod enthält oder ein ungesättigter oder aromatischer Kohlenwasserstoff oder ein gesättigter oder ungesättigter Aldehyd oder ein Keton ist. Mehr bevorzugte Elemente sind Stickstoff, Sauerstoff, Phosphor, Schwefel, Chlor, Brom; insbesondere bevorzugt sind Stickstoff, Sauerstoff, Phosphor und Schwefel.

Insbesondere handelt es sich bei dem Geruchsstoff um Ammoniak, Schwefelwasserstoff, Schwefeloxide, Stickoxide, Ozon, cyclische oder acyclische Amine, Thiole, Thioether sowie Aldehyde, Ketone, Ester, Ether, Säuren oder Alkohole. Besonders bevorzugt sind Ammoniak, Schwefelwasserstoff, organische Säuren (bevorzugt Essigsäure, Propionsäure, Buttersäure, Isobuttersäure, Valeriansäure, Isovaleriansäure, Capronsäure, Heptylsäure, Laurinsäure, Pelargonsäure) sowie cyclische oder acyclische Kohlenwasserstoffe, die Stickstoff oder Schwefel enthalten sowie gesättigte oder ungesättigte Aldehyde, wie Hexanal, Heptanal, Oktanal, Nonanal, Decanal, Octenal oder Nonenal und insbesondere flüchtige Aldehyde wie Butyraldehyd, Propionaldehyd, Acetaldehyd und Formaldehyd und weiterhin Treibstoffe wie Benzin, Diesel (Inhaltsstoffe).

Bei den Geruchsstoffen kann es sich auch um Riechstoffe, die beispielsweise zur Herstellung von Parfümen verwendet werden, handeln. Beispielhaft seien als Riechstoffe oder Öle, die solche Riechstoffe freisetzen zu nennen: ätherische Öle, Basilikumöl, Geranienöl, Minzöl, Canangabaumöl, Kardamomöl, Lavendelöl, Pfefferminzöl, Muskatöl, Kamillenöl, Eukalyptusöl, Rosmarinöl, Zitronenöl, Limettenöl, Orangenöl, Bergamottenöl, Muskatellersalbeiöl, Korianderöl, Zypressenöl, 1,1-Dimethoxy-2-pherylethan, 2,4-Dimethyl-4-phenyltetrahydrofuran, Dimethyltetrahydrobenzaldehyd, 2,6-Dimethyl-7-octen-2-ol, 1,2-Diethoxy-3,7-dimethyl-2,6-octadien, Phenylacetaldehyd, Rosenoxid, Ethyl-2-methylpentanoat, 1-(2,6,6-Trimethyl-1,3-cyclohexadien-1-yl)-2-buten-1-on, Ethylvanillin, 2,6-Dimethyl-2-octenol, 3,7-Dimethyl-2-octenol, tert-Butylcyclohexylacetat, Anisylacetate, Allylcyclohexyloxyacetat, Ethyllinalool, Eugenol, Cumarin, Ethylacetacetat, 4-Phenyl-2,4,6-trimethyl-1,3-dioxan, 4-Methylen-3,5,6,6-tetramethyl-2-heptanon, Ethyltetrahydrosafranat, Geranylnitril, cis-3-Hexen-1-ol, cis-3-Hexenylacetat, cis-3-Hexenylmethylcarbonate, 2,6-Dimethyl-5-hepten-1-al, 4-(Tricyclo[5.2.1.0]decylidene)-8-butanal, 5-(2,2,3-Trimethyl-3-cyclopentenyl)-3-methylpentan-2-ol, p-tert-Butylalpha-methylhydrozimtaldehyd, Ethyl[5.2.1.0]tricyclodecancarboxylat, Geraniol, Citronellol, Citral, Linalool, Linalylacetat, Jonone, Phenylethanol oder Mischungen hiervon.

Im Rahmen der vorliegenden Erfindung weist ein flüchtiger Geruchsstoff vorzugsweise einen Siedepunkt oder Siedepunktsbereich von weniger als 300 °C auf. Mehr bevorzugt ist der Geruchsstoff eine leicht flüchtige Verbindung oder ein Gemisch. Insbesondere bevorzugt weist der Geruchsstoff einen Siedepunkt oder Siedebereich von weniger als 250 °C, mehr bevorzugt weniger als 230 °C, insbesondere bevorzugt weniger als 200 °C auf.

Bevorzugt sind ebenfalls Geruchsstoffe, die eine hohe Flüchtigkeit aufweisen. Als Maß für die Flüchtigkeit kann der Dampfdruck herangezogen werden. Im Rahmen der vorliegenden Erfindung weist ein flüchtiger Geruchsstoff vorzugsweise einen Dampfdruck von mehr als 0,001 kPa (20°C) auf. Mehr bevorzugt ist der Geruchsstoff eine leicht flüchtige Verbindung oder ein Gemisch. Insbesondere bevorzugt weist der Geruchsstoff einen Dampfdruck von mehr als 0,01 kPa (20°C) auf, mehr bevorzugt einen Dampfdruck von mehr als 0,05 kPa (20°C) auf. Besonders bevorzugt weisen die Geruchsstoffe einen Dampfdruck von mehr als 0,1 kPa (20°C) auf.

### Beispiele

### Beispiel 1 Drucklose Herstellung/Charakterisierung eines Al-BTC-MOFs

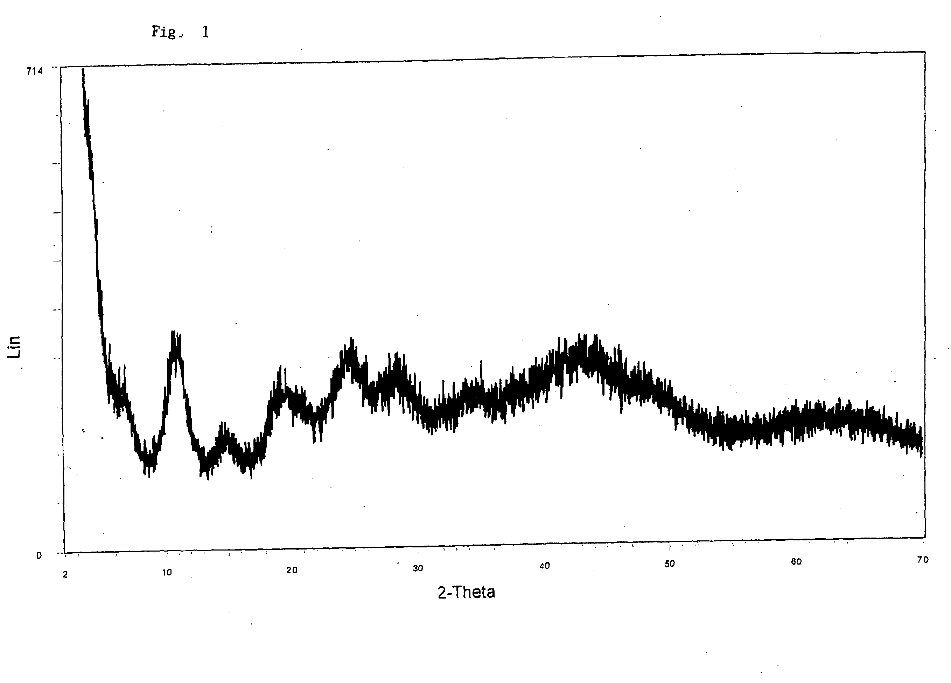

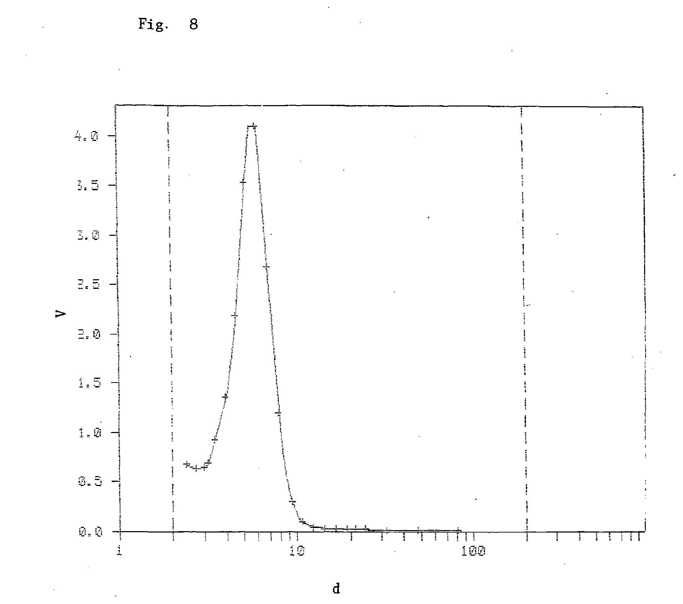

7,8 g 1,3,5-Benzoltricarbonsäure und 22,9 g Al(NO₃)₃*9H₂O werden in 520,5 g DMF in einem Glaskolben suspendiert und 4 Tage bei 130°C unter Rückfluss gerührt. Nach dem Abkühlen wird der Feststoff abfiltriert, mit 2x100 ml DMF und 4x100 ml Methanol gewaschen und im Vakuumtrockenschrank 16 h bei 200°C getrocknet. Anschließend wird die Probe 3 Tage lang in einem Muffelofen (100 l/h Luft) bei 330°C nachbehandelt (Aufheizen mit ca. 75 °C/h). Es werden 8,4 g eines Al-BTC-MOFs erhalten mit einer Oberfläche von 1791 m²/g (mit N₂ nach Langmuir). Das Röntgendiffraktogramm (XRD) ist in Fig. 1 gezeigt. Für sämtliche Diffraktogramme wird die Probe ungemörsert unter einer N₂-Glocke präpariert und mit einer Styroflexfolie luftdicht überzogen. Die Probe wird an einem Gerät D5000 der Firma Siemens mit einer Cu-Anode bei einer Schrittbreite von 0,02° und einer Schrittgeschwindigkeit von 3,6 s aufgenommen. Das Diffraktogramm zeigt - untypisch für MOFs - kaum Reflexe und wirkt sehr amorph. Die Porenverteilung ist in Fig. 8 wiedergegeben. Dargstellt is in Fig. 8 das Porenvolumen V (ml/g) als Funktion des Porendurchmessers d (nm). Hier zeigt sich der für MOFs ungewöhnlich hohe Anteil an Mesoporen.

### Beispiel 2 Hydrothermale Herstellung/Charakterisierung eines AI-BTC-MOFs

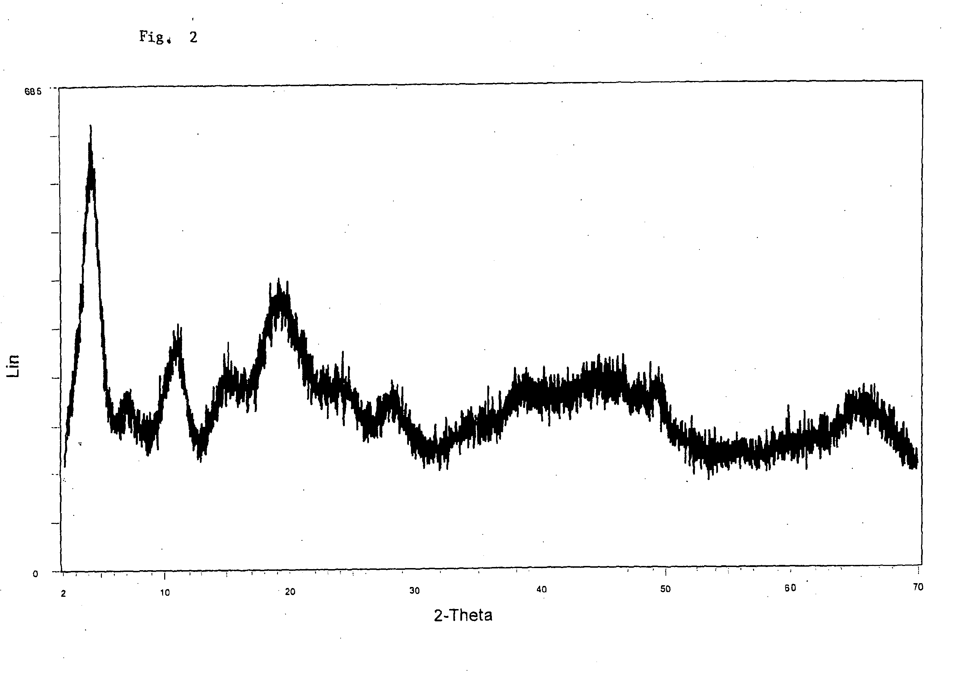

2,46 g 1,3,5-Benzoltricarbonsäure und 8,7 g Al(NO₃)₃*9H₂O werden in 33 ml DMF suspendiert. Das Gemisch wird in einem Berghoff-Autoklaven ("Teflon-Liner") 1 Tag bei 170°C getempert. Nach dem Abkühlen wird der Feststoff abfiltriert, mit DMF und Methanol gewaschen und im Vakuumtrockenschrank 5 h bei 200°C getrocknet. Anschließend wird die Probe 3 Tage lang in einem Muffelofen (100 l/h Luft) bei 330 °C nachbehandelt (Aufheizen mit ca. 75 °C/h). Es werden 2,5 g eines Al-BTC-MOFs erhalten mit einer Oberfläche von 1516 m²/g (mit N₂ nach Langmuir). Das XRD ist in Fig. 2 gezeigt. Das Diffraktogramm zeigt - untypisch für MOFs - kaum Reflexe und wirkt sehr amorph.

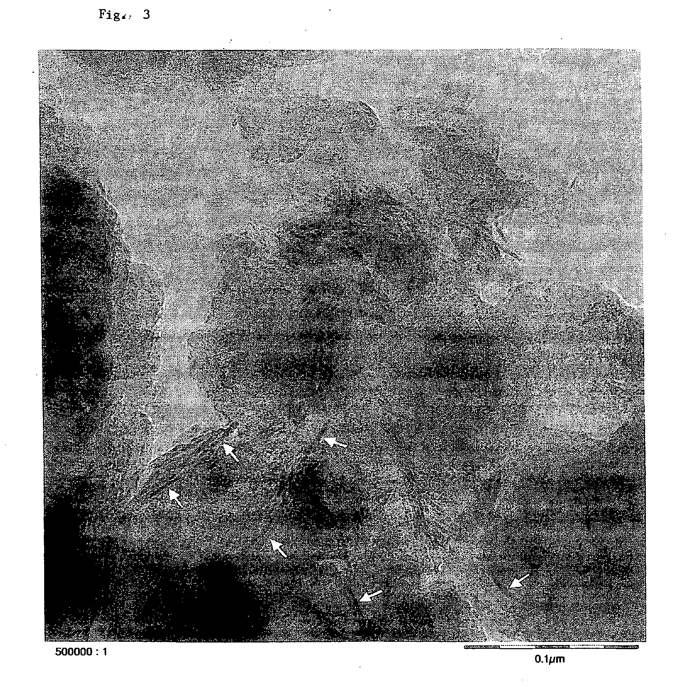

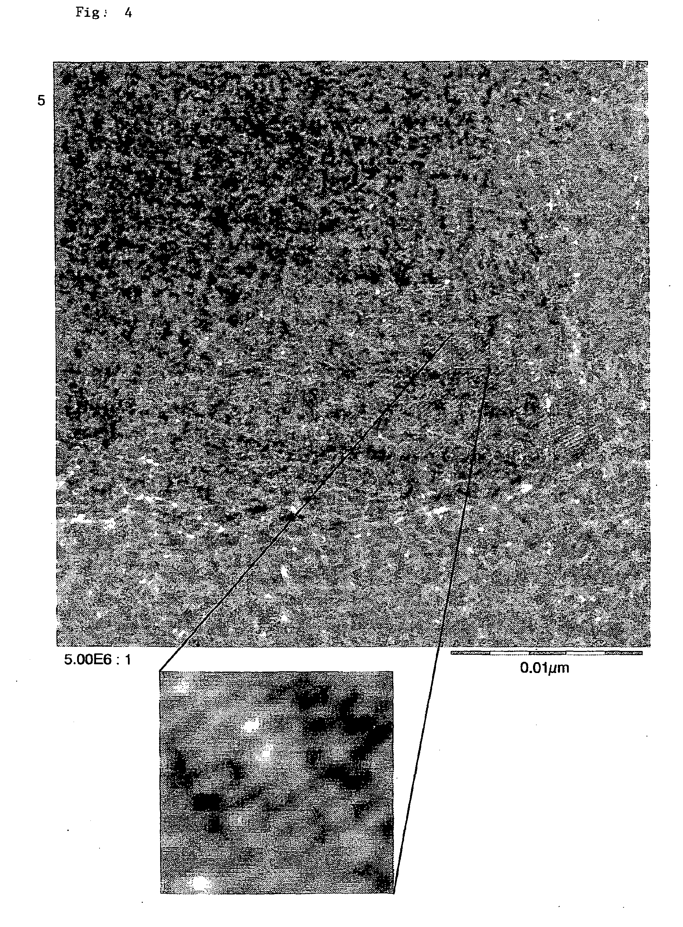

Transmissionselektronenmikroskop (TEM)-Aufnahmen sind in Fig. 3 und Fig. 4 gezeigt. Diese zeigen klar das Vorhandensein von kristallinen Teilstrukturen.

Die TEM-Aufnahme erfolgt, indem die Probe mit etwas Ethanol versetzt und zwischen 2 Glasobjektträgern zerrieben wird. Dann wird diese dünn ausgestrichen und mit einem mit Formvar/Kohle befilmten Präparatträgernetzchen aufgetupft. Die Aufnahmen entstanden mit einem 200 kV FEG-TEM der Firma FEI. Gerätebezeichnung: Tecnai G².

### Beispiel 3 Herstellung/Charakterisierung eines Al-BTC-MOFs

15,6 g 1,3,5-Benzoltricarbonsäure und 45,8 g Al(NO₃)₃*9H₂O werden in 520,5 g DMF in einem Glaskolben suspendiert 4 Tage bei 130°C unter Rückfluss gerührt. Nach dem Abkühlen wird der Feststoff abfiltriert, mit 2x100 ml DMF und 4x100 ml Methanol gewaschen und im Vakuumtrockenschrank 16 h bei 200°C getrocknet. Anschließend wird die Probe 3 Tage lang in einem Muffelofen (100 l/h Luft) bei 330 °C nachbehandelt (Aufheizen mit ca. 75 °C/h). Es werden 17,7 g eines Al-BTC-MOFs erhalten mit einer Oberfläche von 1696 m²/g (mit N₂ nach Langmuir). Das Diffraktogramm (XRD) zeigt im Vergleich zu Beispiel 2 keine signifikanten Unterschiede.

### Beispiel 4 Herstellung/Charakterisierung eines Al-BTC-MOFs

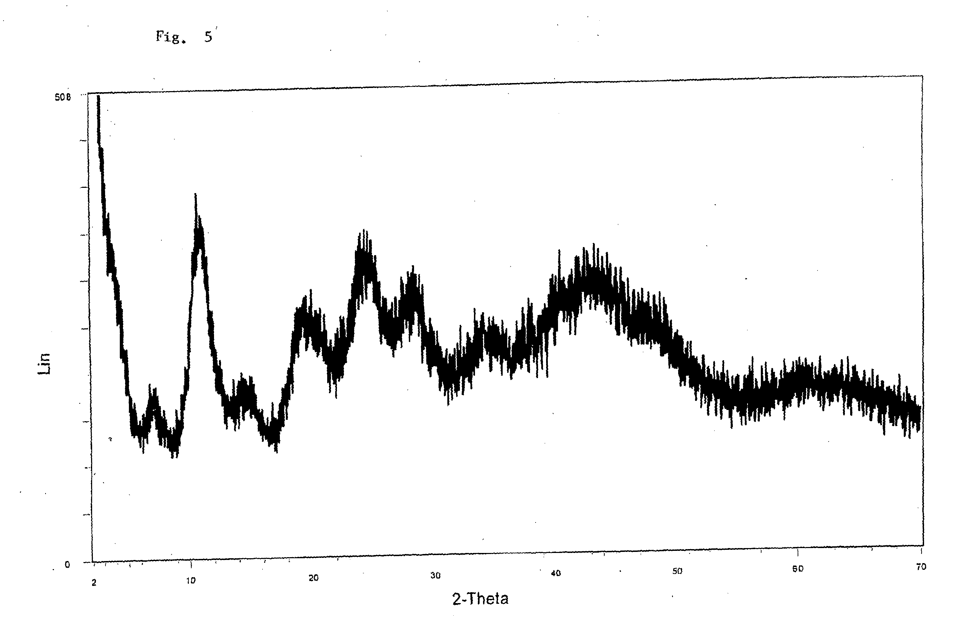

7,8 g 1,3,5-Benzoltricarbonsäure und 14,7 g AlCl₃*6H₂O werden in 520,5 g DMF in einem Glaskolben suspendiert 4 Tage bei 130°C unter Rückfluss gerührt. Nach dem Abkühlen wird der Feststoff abfiltriert, mit 2x100 ml DMF und 4x100 ml Methanol gewaschen und im Vakuumtrockenschrank 16 h bei 200°C getrocknet. Anschließend wird die Probe 3 Tage lang in einem Muffelofen (100 l/h Luft) bei 330 °C nachbehandelt (Aufheizen mit ca. 75 °C/h). Es werden 10,9 g eines Al-BTC-MOFs erhalten mit einer Oberfläche von 1451 m²/g (mit N₂ nach Langmuir). Das Diffraktogramm (XRD) ist in Fig. 5 dargestellt.

### Beispiel 5 Herstellung/Charakterisierung eines Al-BTC-MOFs

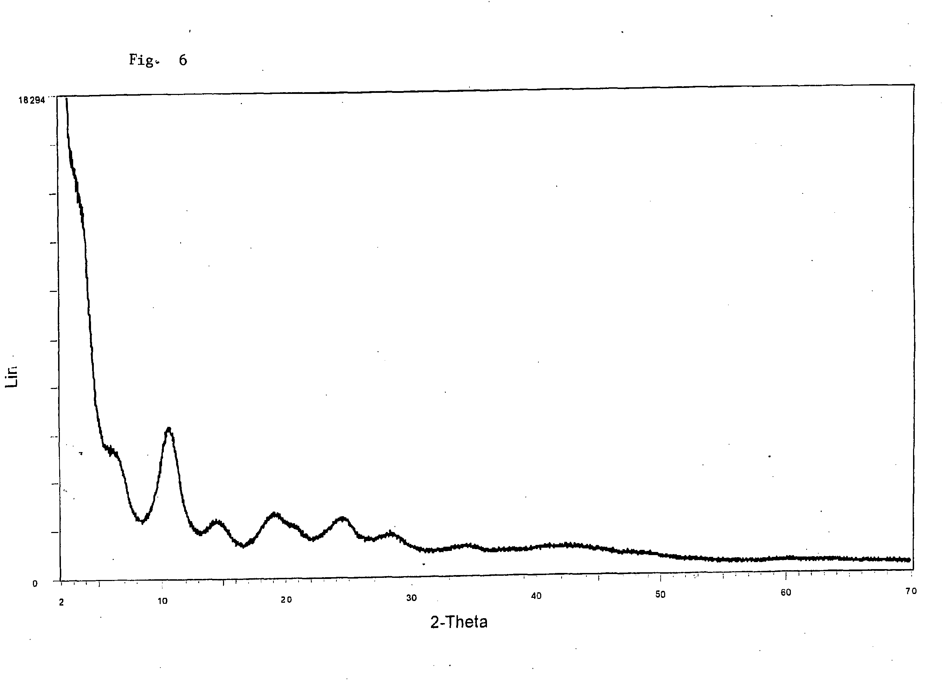

23,1 g 1,3,5-Benzoltricarbonsäure und 29,4 g AlCl₃*6H₂O werden in 520,5 g DMF in einem Glaskolben suspendiert 4 Tage bei 130°C unter Rückfluss gerührt. Nach dem Abkühlen wird der Feststoff abfiltriert, mit 2x100 ml DMF und 4x100 ml Methanol gewaschen und im Vakuumtrockenschrank 16 h bei 200°C getrocknet. Anschließend wird die Probe 3 Tage lang in einem Muffelofen (100 l/h Luft) bei 330 °C nachbehandelt (Aufheizen mit ca. 75 °C/h). Es werden 12,3 g eines Al-BTC-MOFs erhalten mit einer Oberfläche von 2033 m²/g (mit N₂ nach Langmuir). Das Diffraktogramm (XRD) ist in Fig. 6 dargestellt.

### Beispiel 6 Herstellung/Charakterisierung eines Al-BTC-MOFs

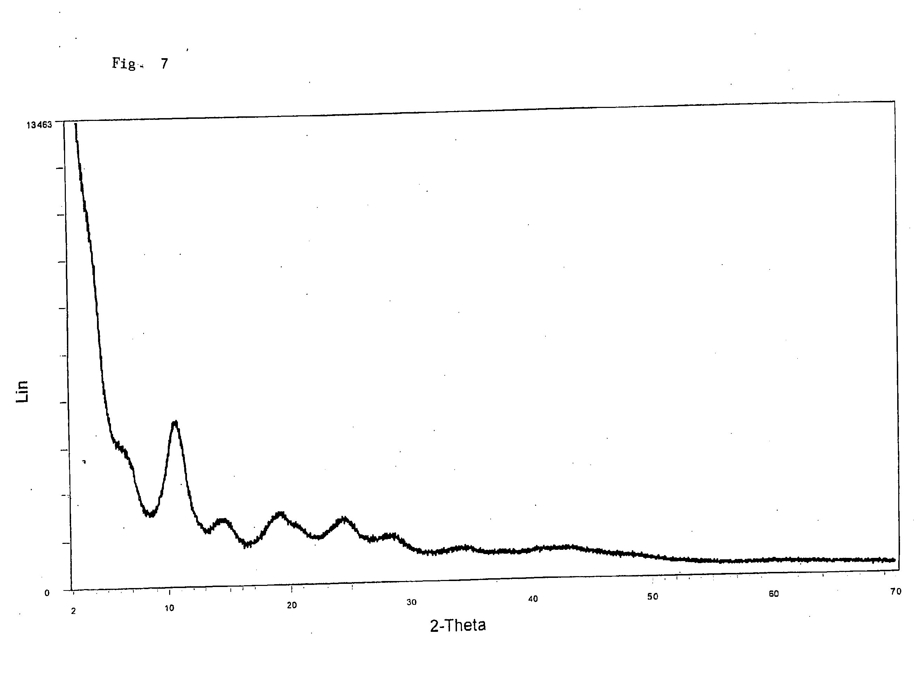

15,6 g 1,3,5-Benzoltricarbonsäure und 29,4 g AlCl₃*6H₂O werden in 520,5 g DMF in einem Glaskolben suspendiert 4 Tage bei 130°C unter Rückfluss gerührt. Nach dem Abkühlen wird der Feststoff abfiltriert, mit 2x100 ml DMF und 4x100 ml Methanol gewaschen und im Vakuumtrockenschrank 16 h bei 200°C getrocknet. Anschließend wird die Probe 3 Tage lang in einem Muffelofen (100 l/h Luft) bei 330 °C nachbehandelt (Aufheizen mit ca. 75 °C/h). Es werden 20,1 g eines Al-BTC-MOFs erhalten mit einer Oberfläche von 1898 m²/g (mit N₂ nach Langmuir). Das Diffraktogramm (XRD) ist in Fig. 7 dargestellt.

### Beispiel 7 Speicherung von Wasserstoff in einem Al-BTC-MOFs

314 mg des Gerüstmaterials aus Beispiel 1 werden zunächst bei 200°C evakuiert. Anschließend erfolgt die Wasserstoffaufnahme mit Hilfe des Gerätes Quantachrome Autosorb 1 bei 77 K. Es ergab sich eine Wasserstoffaufnahme bei p/p₀ = 6,5 * 10⁻³ von ca. 100 ml/g.

## Patentansprüche

1. Poröses metallorganisches Gerüstmaterial enthaltend mindestens eine an mindestens ein Metallion koordinativ gebundene mindestens zweizähnige organische Verbindung, wobei das mindestens eine Metallion Al^{III} ist und es sich bei der mindestens einen mindestens zweizähnigen organischen Verbindung um einen sechsgliedrigen aromatischen Kohlenwasserstoffring A, bei dem ein oder mehrere Ringkohlenstoffatome durch Stickstoff ersetzt sein können und der drei Substituenten X sowie gegebenenfalls einen oder mehrere Substituenten ausgewählt aus der Gruppe bestehend aus R, NRR', OR, SR, F, Cl, und Br aufweist, wobei R, R' unabhängig voneinander Wasserstoff, optional mit einem oder mehreren Fluoratomen substituiertes Methyl oder optional mit einem oder mehreren Fluoratomen substituiertes Ethyl sind und jedes X jeweils unabhängig C(=O)O⁻, C(=S)O⁻, C(=O)S⁻, C(=S)S⁻ oder deren protonierte Form ist, handelt, wobei das metallorganische Gerüstmaterial in Pulverform eine spezifische Oberfläche nach Langmuir von mindestens 800 m²/g aufweist und wobei mindestens 50% des Porenvolumens durch Poren mit einem Porendurchmesser von 2 bis 10 nm gebildet wird.

2. Gerüstmaterial nach Anspruch 1, **dadurch gekennzeichnet, dass** A Benzol, Pyridin, Pyridazin, Pyrimidin, Pyrazin oder Triazin ist.

3. Gerüstmaterial nach Anspruch 1 oder 2, **dadurch gekennzeichnet, dass** die mindestens eine mindestens zweizähnige organische Verbindung 1,3,5-, 1,2,3-, 1,2,4-Benzoltricarboxylat oder deren protonierte Form ist.

4. Gerüstmaterial nach einem der Ansprüche 1 bis 3, **dadurch gekennzeichnet, dass** das Gerüstmaterial einen molaren Anteil an der mindestens einen mindestens zweizähnigen organischen Verbindung von mindestens 50 % der Gesamtmenge der organischen Verbindungen aufweist.

5. Gerüstmaterial nach einem der Ansprüche 1 bis 4, **dadurch gekennzeichnet, dass** der am häufigsten auftretende Porendurchmesser im Bereich von 3 bis 9 nm liegt.

6. Gerüstmaterial nach einem der Ansprüche 1 bis 5, **dadurch gekennzeichnet, dass** das Porenvolumen des Gerüstmaterials mindestens 0,8 mg/l aufweist.

7. Gerüstmaterial nach einem der Ansprüche 1 bis 6, **dadurch gekennzeichnet, dass** dieses in Pulverform eine spezifische Oberfläche nach Langmuir von mindestens 1000 m²/g aufweist.

8. Verfahren zur Herstellung eines Gerüstmaterials nach einem der Ansprüche 1 bis 7 den Schritt enthaltend
Umsetzung mindestens einer Metallverbindung mit mindestens einer mindestens zweizähnigen organischen Verbindung, die koordinativ an das Metallion binden kann, in N,N-Dimethylformamid, N,N-Diethylformamid, N-Methylpyrolidon oder Mischungen davon, wobei das Metall Al^{III} ist und wobei es sich bei der mindestens einen mindestens zweizähnigen organischen Verbindung um einen sechsgliedrigen aromatischen Kohlenwasserstoffring A, bei dem ein oder mehrere Ringkohlenstoffatome durch Stickstoff ersetzt sein können und der drei Substituenten X sowie gegebenenfalls einen oder mehrere Substituenten ausgewählt aus der Gruppe bestehend aus R, NRR', OR, SR, F, Cl, und Br aufweist, wobei R, R' unabhängig voneinander Wasserstoff, optional mit einem oder mehreren Fluoratomen substituiertes Methyl oder optional mit einem oder mehreren Fluoratomen substituiertes Ethyl sind und jedes X jeweils unabhängig C(=O)O⁻, C(=S)O⁻, C(=O)S⁻, C(=S)S⁻ oder deren protonierte Form ist, handelt.

9. Verfahren nach Anspruch 8, **dadurch gekennzeichnet, dass** nach der Umsetzung das gebildete Gerüstmaterial mit einem organischen Lösemittel nachbehandelt und/oder calciniert wird.

10. Verfahren nach Anspruch 7 oder 8, **dadurch gekennzeichnet, dass** Al^{III} durch anodische Oxidation für die Umsetzung bereitgestellt wird.

11. Verwendung eines Gerüstmaterials nach einem der Ansprüche 1 bis 7 zur Aufnahme mindestens eines Stoffes zu dessen Speicherung, Abtrennung oder kontrollierten Abgabe.

12. Verwendung nach Anspruch 11 zur Wasserstoffspeicherung.

## Claims

1. A porous metal-organic framework comprising at least one at least bidentate organic compound coordinated to at least one metal ion, wherein the at least one metal ion is Al^{III} and the at least one at least bidentate organic compound is a six-membered aromatic hydrocarbon ring A in which one or more ring carbons may be replaced by nitrogen and which has three substituents X and optionally one or more substituents selected from the group consisting of R, NRR', OR, SR, F, Cl and Br, where R, R' are each, independently of one another, hydrogen, methyl which may optionally be substituted by one or more fluorine atoms or ethyl which may optionally be substituted by one or more fluorine atoms and each X is, independently of the others, C(=O)O⁻, C(=S)O⁻, C(=O)S⁻, C(=S)S- or a protonated form thereof, the metal-organic framework in powder form has a specific surface area determined by the Langmuir method of at least 800 m²/g and at least 50% of the pore volume is formed by pores having a pore diameter of from 2 to 10 nm.

2. The framework according to claim 1, wherein A is benzene, pyridine, pyridazine, pyrimidine, pyrazine or triazine.

3. The framework according to claim 1 or 2, wherein the at least one at least bidentate organic compound is 1,3,5-, 1,2,3-, 1,2,4-benzenetricarboxylate or a protonated form thereof.

4. The framework according to any of claims 1 to 3 which has a mole fraction of the at least one at least bidentate organic compound of at least 50% of the total amount of the organic compounds.

5. The framework according to any of claims 1 to 4, wherein the most frequently occurring pore diameter is in the range from 3 to 9 nm.

6. The framework according to any of claims 1 to 5 whose pore volume is at least 0.8 mg/l.

7. The framework according to any of claims 1 to 6 which in powder form has a specific surface area determined by the Langmuir method of at least 1000 m²/g.

8. A process for preparing a framework according to any of claims 1 to 7, which comprises the step reaction of at least one metal compound with at least one at least bidentate organic compound which can coordinate to the metal ion in N,N-dimethylformamide, N,N-diethylformamide, N-methylpyrrolidone or mixtures thereof, wherein the metal is Al^{III} and the at least one at least bidentate organic compound is a six-membered aromatic hydrocarbon ring A in which one or more ring carbons may be replaced by nitrogen and which has three substituents X and optionally one or more substituents selected from the group consisting of R, NRR', OR, SR, F, Cl and Br, where R, R' are each, independently of one another, hydrogen, methyl which may optionally be substituted by one or more fluorine atoms or ethyl which may optionally be substituted by one or more fluorine atoms and each X is, independently of the others, C(=O)O⁻, C(=S)O⁻, C(=O)S⁻, C(=S)S⁻ or a protonated form thereof.

9. The process according to claim 8, wherein the framework formed is after-treated with an organic solvent and/or calcined after the reaction.

10. The process according to claim 7 or 8, wherein Al^{III} is made available for the reaction by means of anodic oxidation.

11. The use of a framework according to any of claims 1 to 7 for the uptake of at least one substance for the purposes of its storage, separation or controlled release.

12. The use according to claim 11 for the storage of hydrogen.

## Revendications

1. Matériau de structure organométallique mésoporeux contenant au moins un composé organique au moins bidentate lié de façon coordinative à au moins un ion métallique, l'au moins un ion métallique étant de l'Al^{III} et l'au moins un composé organique au moins bidentate étant un noyau d'hydrocarbure aromatique A à six chaînons, dans lequel un ou plusieurs atomes de carbone nucléaire peuvent être remplacés par de l'azote et qui comporte trois substituants X, ainsi que le cas échéant un ou plusieurs substituants choisis dans le groupe composé de R, NRR', OR, SR, F, Cl, et Br, R, R' étant indépendamment l'un de l'autre de l'hydrogène, en option du méthyle substitué par un ou plusieurs atomes de fluor ou en option de l'éthyle substitué par un ou plusieurs atomes de fluor et chaque X étant à chaque fois indépendamment C(=O)O⁻, C(=S)O⁻, C(=O)S⁻, C(=S)S⁻ ou leur forme protonée, le matériau à structure organométallique sous la forme pulvérulente présentant une surface spécifique selon Langmuir d'au moins 800 m²/g et au moins 50 % du volume poreux étant formé par des pores d'un diamètre de 2 à 10 nm.

2. Matériau de structure selon la revendication 1, **caractérisé en ce que** A est du benzène, de la pyridine, de la pyridazine, de la pyrimidine, de la pyrazine ou de la triazine.

3. Matériau de structure selon la revendication 1 ou 2, **caractérisé en ce que** l'au moins un composé organique au moins bidentate est du tricarboxylate de 1,3,5-, 1,2,3-, 1,2,4-benzène ou leur forme protonée.

4. Matériau de structure selon l'une quelconque des revendications 1 à 3, **caractérisé en ce que** le matériau de structure présente une part moléculaire de l'au moins un composé organique au moins bidendate d'au moins 50 % de la quantité totale du composé organique.

5. Matériau de structure selon l'une quelconque des revendications 1 à 4, **caractérisé en ce que** le diamètre de pores le plus fréquent est de l'ordre de 3 à 9 nm.

6. Matériau de structure selon l'une quelconque des revendications 1 à 5, **caractérisé en ce que** le volume poreux du matériau de structure présente au moins 0,8 mg/l.

7. Matériau de structure selon l'une quelconque des revendications 1 à 6, **caractérisé en ce que** ce dernier présente sous la forme pulvérulente une surface spécifique selon Langmuir d'au moins 1000 m²/g.

8. Procédé de production d'un matériau de structure selon l'une quelconque des revendications 1 à 7, comportant l'étape de
transformation d'au moins un composé métallique avec au moins un composé organique au moins bidendate, qui peut se lier de façon coordinative à l'ion métallique, dans du N,N-diméthylformamide, du N,N-diéthylformamide, du N-méthylpyrrolidone ou des mélanges de ces derniers, le métal étant de l'Al^{III} et l'au moins un composé organique au moins bidentate étant un noyau d'hydrocarbure aromatique A à six chaînons, dans lequel un ou plusieurs atomes de carbone nucléaire peuvent être remplacés par de l'azote et qui comporte trois substituants X, ainsi que le cas échéant un ou plusieurs substituants choisis dans le groupe composé de R, NRR', OR, SR, F, Cl, et Br, R, R' étant indépendamment l'un de l'autre de l'hydrogène, en option du méthyle substitué avec un ou plusieurs atomes de fluor ou en option de l'éthyle substitué par un ou plusieurs atomes de fluor et chaque X étant à chaque fois indépendamment C(=O)O⁻, C(=S)O⁻, C(=O)S⁻, C(=S)S⁻ ou leur forme protonée.

9. Procédé selon la revendication 8, **caractérisé en ce qu'**on traite ultérieurement et/ou on fait calciner avec un solvant organique le matériau de structure formé.

10. Procédé selon la revendication 7 ou 8, **caractérisé en ce que** pour la transformation, on met à disposition de l'Al^{III} par oxydation anodique.

11. Utilisation d'un matériau de structure selon l'une quelconque des revendications 1 à 7 pour recevoir au moins une substance, pour son stockage, sa séparation ou sa distribution contrôlée.

12. Utilisation selon la revendication 11 pour le stockage d'hydrogène.
